# EUROPÄISCHE PATENTSCHRIFT

(19) Europäisches Patentamt
European Patent Office
Office européen des brevets

(11) Veröffentlichungsnummer : **0 341 202 B1**

(12) **EUROPÄISCHE PATENTSCHRIFT**

(45) Veröffentlichungstag der Patentschrift :
**29.07.92 Patentblatt 92/31**

(51) Int. Cl.⁵ : **A61K 47/22, A61L 15/44**

(21) Anmeldenummer : **89810285.0**

(22) Anmeldetag : **13.04.89**

(54) **Transdermale Monolithsysteme.**

(30) Priorität : **22.04.88 CH 1524/88**

(43) Veröffentlichungstag der Anmeldung :
**08.11.89 Patentblatt 89/45**

(45) Bekanntmachung des Hinweises auf die
Patenterteilung :
**29.07.92 Patentblatt 92/31**

(84) Benannte Vertragsstaaten :
**AT BE CH DE ES FR GB GR IT LI LU NL SE**

(56) Entgegenhaltungen :
**EP-A- 0 069 385**
**EP-A- 0 147 146**

(56) Entgegenhaltungen :
**EP-A- 0 148 391**
**EP-A- 0 150 428**
**EP-A- 0 169 364**
**EP-A- 0 186 019**
**EP-A- 0 204 968**
**EP-A- 0 279 982**

(73) Patentinhaber : **CIBA-GEIGY AG**
**Klybeckstrasse 141**
**CH-4002 Basel (CH)**

(72) Erfinder : **Sinnreich, Joel, Dr.**
**Hohe Winde Strasse 98**
**CH-4059 Basel (CH)**
Erfinder : **Fankhauser, Peter, Dr.**
**Hauptstrasse 65**
**CH-4107 Ettingen (CH)**

Jouve, 18, rue Saint-Denis, 75001 PARIS

**Beschreibung**

Die Erfindung betrifft ein pharmazeutisches Pflaster für die transdermale Verabreichung von hautpermeationsfähigen Wirkstoffen, ein Verfahren zur Herstellung dieses Pflasters sowie die therapeutische Verwendung dieses Pflasters bei der Prävention und Behandlung diverser Zustände und Krankheiten.

Die topische Applikation von Wirkstoffen unter Verwendung von transdermalen therapeutischen Systemen gilt als besonders vorteilhafte Applikationsform für solche Fälle, in denen die orale bzw. eine andere parenterale Applikationsform nachteilig wäre und beispielsweise zu Unverträglichkeiten oder Nebenwirkungen des verabreichten Wirkstoffs führt. Insbesondere ist die Applikation mit transdermalen therapeutischen Systemen vor allem dann günstig, wenn eine kontinuierliche Freisetzung der Wirkstoffkomponente über einen längeren Zeitraum beabsichtigt ist, oder Wirkstoffe unter Umgehung des gastrointestinalen Trakts systemisch wirken sollen, deren Stabilität im gastrointestinalen Trakt unzureichend wäre.

Wegen der geringen Durchlässigkeit der Haut, insbesondere der äusseren Hornhautschicht, müssen die transdermal zu applizierenden Wirkstoffe folgendes Anforderungsprofil erfüllen:

1. Sie müssen trotz der Sperrfunktion der äusseren Hornschicht ausreichende Hautpermeabilität besitzen, um in den Blutkreislauf zu gelangen;

2. Sie müssen gute Hautverträglichkeit aufweisen;

3. Sie müssen ausserdem für eine prophylaktische oder therapeutische Daueranwendung oder eine Substitutionstherapie geeignet sein (siehe auch H. Asche Pharma International 4 (P), 1984, S. 162).

Dieses Anforderungsprofil begrenzt die Auswahl der zur Verfügung stehenden transdermal applizierbaren Wirkstoffe, so dass entsprechende therapeutische Systeme nur mit wenigen Wirkstoffen bisher Verwendung in der Therapie gefunden haben, z.B. mit den Wirkstoffen Scopolamin, Nitroglycerin, Estradiol oder Clonidin.

Es ist bekannt, dass man die Permeationsfähigkeit von systemischen Wirkstoffen mit bestimmten Hautpenetrationsmitteln ("flux enhancer"), z.B. mit Dimethylsulfoxid, Dimethylformamid oder Methyl-n-dodecylsulfoxid, siehe U.S. Patentschrift 3,257,864, 1-n-Dodecylazacycloheptan-2-on, siehe U.S. Patentschrift 4,316,893, oder Aethanol, siehe Deutsche Offenlegungsschrift 32 05 258, steigern kann. Die Eignung von Eucalyptol und Gemischen von Eucalyptol mit anderen Penetrationsmitteln, wie N-Methyl-2-pyrrolidon, insbesondere im Mischungsverhältnis von 1:1, zur Steigerung der Permeationsfähigkeit von Wirkstoffen in topischen Präparaten wie Cremen, Salben, Pasten und Lotionen ist in der Europäischen Patentanmeldung 69 385 beschrieben.

In solchen topischen Präparaten kann das geruchsintensive Eucalyptol, welches 70-100% 1,8-Cineal enthält, offen austreten. Ausserdem sind diese nur kurze Zeit applizierbar, müssen daher häufig erneuert werden und erlauben generell nur eine ungenaue Dosierung des Wirkstoffs.

Der vorliegenden Erfindung liegt die Aufgabe zugrunde, eine verbesserte Darreichungsform für die topische Applikation von Wirkstoffen in Form eines transdermalen therapeutischen Systems mit dem Hautpenetrationsmittel Eucalyptol herzustellen.

Diese Aufgabe wird durch die vorliegende Erfindung gelöst, welche ein transdermales therapeutisches System mit chemisch reinem 1,8-Cineol als Hautpenetrationsmittel in einer festen Matrix und günstigen Stabilitäts- und Abgabeeigenschaften betrifft.

Das erfindungsgemässe transdermale therapeutische System hat die Form eines mehrschichtigen Pflasters mit folgenden Bestandteilen:

a) eine für die Bestandteile der Haftklebeschicht b) undurchlässige Deckschicht;

b) eine Wirkstoff-abgabefähige Haftklebeschicht aus permeablem, hautverträglichem, polymerem Material, welche mindestens einen permeationsfähigen Wirkstoff, eine Kombination aus chemisch reinem 1,8-Cineol (Reinheitsgrad höher als 99 %) und N-Methyl-2-pyrrolidon als zusätzliches Hautpenetrationsmittel und gegebenenfalls weitere pharmazeutische Hilfsstoffe enthält, und

c) eine von der Haftklebeschicht b) abziehbare Schutzfolie.

Die weiter vorn und im folgenden verwendeten Begriffe und Definitionen haben im Rahmen der Beschreibung der vorliegenden Erfindung vorzugsweise die folgenden Bedeutungen:

Die Deckschicht (a) besteht aus einem Material oder einer Kombination von Materialien, die für die Bestandteile der Haftklebeschicht (b), insbesondere flüssige Bestandteile der Formulierung, undurchlässig sein müssen. Sie dient als äussere Schutzschicht des Systems. Zur Herstellung dieser Deckschicht können Hoch- oder Niederdruckpolymere wie polyäthylen, polypropylen, polyvinylchlorid, polyäthylenterephthalat oder auch Celluloseacetat oder Vinylacetat-Vinylchlorid-Copolymere und Kombinationen, insbesondere Verbundfolien daraus, verwendet werden. Bevorzugt ist eine undurchlässige, flexible Deckschicht, welche sich der Ausformung der betreffenden Körperpartie anpasst, worauf das Pflaster angebracht ist.

Die Haftklebeschicht b) befindet sich zwischen der Deckschicht a) und der Folie c) und besteht aus einem hautverträglichen, polymeren Material in Form einer Matrix, welche den Wirkstoff oder eine Wirkstoffkombination und zur Verstärkung der Hautdurchlässigkeit eine Kombination aus chemisch reinem 1,8-Cineol und N-Methyl-2-pyrrolidon als zusätzlichem Hautpenetrationsmittel und gegebenenfalls weitere Hilfsstoffe enthält.

Geeignete polymere'Materialien sind sowohl für den Wirkstoff als auch für die Kombination aus 1,8-Cineol mit dem zusätzlichen penetrationsmittel permeabel und besitzen neben Hautverträglichkeit ausreichende Klebefähigkeit, so dass das therapeutische System mindestens einen Tag lang auf der Haut befestigt bleibt und sich danach ohne erhöhten Kraftaufwand entfernen lässt. Ausserdem sind geeignete polymere Materialien trotz der Lösungsmitteleigenschaften des chemisch reinen 1,8-Cineols formstabil.

Solche polymeren Materialien sind z.B. Siliconkautschuk (Silicone), z.B. lineare Polysiloxane, worin die Siliciumatome in der Siloxankette durch zwei gleiche oder verschiedene Alkyl-, z.B. Methyl- oder Aethyl-, Aryl-, z.B. Phenyl-, Alkenyl-, z.B. Vinyl- oder Allyl-, Alkylaryl-, z.B. Tolyl- oder Xylyl-, oder Aralkyl-, z.B. Benzylreste, und die terminalen Siliciumatome durch drei der genannten organischen Reste substituiert sind. Die Herstellung dieser Silicone ist in den U.S. Patentschriften 2,541,137, 2,723,966, 2,863,846, 2,890,188, 2,927,907, 3,002,951 und 3,035,016 beschrieben, wobei bei Raumtemperatur vulkanisierbare Silicone bevorzugt sind.

Geeignete polymere Materialien sind ferner hydrophile Polymerisate von Monoestern ungesättigter Carbonsäuren wie Acrylsäure oder Methacrylsäure, z.B. deren Polyhydroxyäthylacrylate oder Polyhydroxyäthylmethacrylate, deren Herstellung in den U.S. Patentschriften 2,976,576 und 3,220,960 beschrieben ist, sowie Copolymere aus wasserlöslichen aliphatischen oder zyklischen Vinylamiden, z.B. Poly-N-vinyl-methylacetamid, -äthylacetamid, -methylpropionamid, -äthylpropionamid, -methylisobutyramid, -2-pyrrolidon, -2-piperidon, -epsilon-caprolactam, -5-methyl-2-pyrrolidon oder -3-methyl-2-pyrrolidon, insbesondere poly-N-vinylpyrrolidon mit einer mittleren Molmasse von 10 000 - 360 000, mit wasserlöslichem Polyvinylacetat oder Polyvinylalkohol mit unterschiedlichem Acetatgehalt, z.B. Polyvinylacetat mit einer Molmasse von 5 000 bis 400 000 oder Polyvinylalkohol mit einem Hydrolysegrad von 85-98 % und einem Polymerisationsgrad von 500 - 2 500.

Bevorzugt sind natürlicher oder synthetischer Kautschuk, z.B. Polyisopren, Butadien-1,4-Polymerisat oder Polyisobutylen und Gemische davon. Insbesondere sind Gemische aus verschiedenen Polyisobutylenen mit unterschiedlichen Molekulargewichtsbereichen von $1,0 \times 10^3$ bis $5,0 \times 10^4$ und $1,0 \times 10^5$ bis $5,0 \times 10^6$ bevorzugt.

Der transdermal zu applizierende Wirkstoff wird vom erfindungsgemässen transdermalen System in therapeutisch wirksamer Dosis durch die Haut aufgenommen. Dabei lassen sich sämtliche systemisch wirksame, permeationsfähige Arzneimittel verwenden, welche von der mit dem Pflaster versehenen Körperoberfläche absorbiert werden, wobei man bekannte Dosisangaben und Anwendungsvorschriften einhält. Zu verwendende Dosismengen hängen vom zu applizierenden Wirkstoff ab. Geeignete systemisch wirksame Arzneimittel sind beispielsweise antibakterielle Wirkstoffe wie Penicilline, Tetracycline, Oxytetracycline, Chlortetracycline, Chloramphenicol oder Sulfonamide, Sedativa und/oder Hypnotika wie Pentabarbital-Natrium, Phenobarbital, Secobarbital-Natrium, Codein, Alpha-Bromisovalerylharnstoff, Carbromal oder Natriumphenobarbital, Psychostimulanzien wie 3-(2-Aminopropyl)indolacetat oder 3-(2-Aminobutyl)indolacetat, Antihypertensiva wie Reserpin oder Clonidin, Beruhigungsmittel wie Chlorpromazinhydrochlorid oder Thiopropazathydrochlorid, Hormone wie Adrenocorticosteroide, z.B. 6α-Methylprednisolen, androgene Steroide, z.B. Methyltestosteron und Fluoxymesteron, östrogene Steroide, z.B. Oestron, 17β-Oestradiol und Aethinylöstradiol, Progesteron oder Norethindron, Kombinationen aus Oestrogenen mit synthetischen Gestagenen oder Progesteronen z.B. 17β-Oestradiol mit Norethisteron-17-acetat oder 17β-Oestradiol mit Progesteron, Antipyretika wie Acetylsalicylsäure, Morphin und andere Analgetika auf Morphinbasis, gefässerweiternde Mittel, z.B. Nitroglycerin oder Isosorbiddinitrat, Herzglycoside wie Digitoxin oder Ouabain, Beta-Blocker wie Propranolol, Oxprenolol oder Metoprolol, Anticholinergika wie Atropin, Methscopolaminbromid, Scopolamin, Hyoscyamin oder Methscopolamin kombiniert mit Phenobarbital, Antimalariamittel wie 4-Aminochinoline, 9-Aminochinoline oder Pyrimethamin, Entwöhnungsmittel zur Beseitigung von Suchtgefahren, z.B. Nicotin zur Raucherentwöhnung, Broncholytika wie Formoterol, Lokalanästhetika wie Tetracain, Nupercain oder Lidocain.

Besonders bevorzugt sind in der Haftklebeschicht b) Nitroglycerin, Scopolamin, Formoterol, 17β-Oestradiol, Progesteron, 17β-Oestradiol kombiniert mit Norethisteronacetat oder Progesteron oder Lidocain als permeationsfähige Wirkstoffe vorhanden.

Die genannten Wirkstoffe, insbesondere die bevorzugten Wirkstoffe, können in der Haftklebeschicht b) in freier Form, z.B. als Säuren oder als Basen, oder als pharmazeutisch annehmbares Salz, z.B. als Chlorid, Bromid, Acetat, Fumarat, Maleat, Succinat oder Lactat, vorhanden sein.

1,8-Cineol ist in Eukalyptusölen, terpentinhaltigen ätherischen Oelen, als Hauptbestandteil enthalten (mehr als 70 %) und aus Blättern, Wurzeln oder Rinde von Eukalyptuspflanzen der Spezies Eucalyptus globulus (Gemeiner Eukalyptusbaum), Eucalyptus maculata, Eucalyptus cladocalyx oder Eucalyptus sideroxylon isolierbar. Durch übliche Reinigungsverfahren, z.B. Rektifizierung, kann dieses Eukalyptusöl zu chemisch reinem 1,8-Cineol (Gehalt mehr als 99 %) weiterverarbeitet werden. In der Haftklebeschicht b) ist dieses chemisch reine 1,8-Cineol mit einem zusätzlichen Hautpenetrationsmittel enthalten.

Hautpenetrationsmittel sind in der Fachliteratur u.a. auch unter Begriffen wie Permeationsverbesserer, dermale Absorptionsmittel oder als Mittel zur Verstärkung der Hautdurchlässigkeit (flux enhancer) bekannt geworden. Solche Mittel haben die Eigenschaft, den Durchfluss von an sich hautundurchlässigen Wirkstoffen durch die Haut, insbesondere die als Barriere wirkende Hornhautschicht (stratum corneum), zu ermöglichen bzw. die Permeationsfähigkeit von Wirkstoffen zu verstärken und dabei die permeation und Aufnahme von therapeutisch wirksamen Mengen des zu applizierenden Wirkstoffs zu ermöglichen.

Durch Verwendung der Kombination von chemisch reinem 1,8-Cineol, mit dem zusätzlichen Hautpenetrationsmittel N-Methyl-2-pyrrolidon wird die Abgabemenge und Abgabegeschwindigkeit (Abgabemenge pro Zeiteinheit) des in der Haftklebeschicht b) enthaltenen Wirkstoffs bzw. einer dort vorhandenen Wirkstoffkombination an die Haut und die entsprechende Aufnahme (bzw. Aufnahmegeschwindigkeit) durch die Haut beträchtlich gesteigert. So werden in einem Zeitraum bis zu 24 Stunden nach Anbringen des Systems weitgehend konstante Mengen des zu applizierenden Wirkstoffs an die Haut abgegeben. Neben dem Wirkstoff selbst bzw. einer Wirkstoffkombination werden ausreichende Mengen der im System enthaltenen penetrationsmittel abgegeben, so dass eine Verstärkung der Hauptpermeabilität des Wirkstoffs und Durchlässigkeit der Haut erfolgt und so die Aufnahme ausreichender Wirkstoffmengen gewährleistet ist.

Bevorzugt ist die Kombination aus 5-9,5 Gewichtsteilen 1,8-Cineol und entsprechend 5-0,5 Gewichtsteilen (bezogen auf 10 Gewichtsteile) N-Methyl-2-pyrrolidon. Solche Kombinationen haben gegenüber reinem N-Methyl-2-pyrrolidon oder 1,8-Cineol den Vorteil, dass diese sich mit dem polymeren Material der Haftklebeschicht b), z.B. polyisobutylengemischen, zu homogenen und abgabefähigen Matrixsystemen mit ausreichender Klebeeigenschaft verarbeiten lassen.

Besonders bevorzugt ist die Kombination von ca. 9 Gewichtsteilen chemisch reinem 1,8-Cineol und 1 Gewichtsteil N-Methyl-2-pyrrolidon.

Der Haftklebeschicht b) können gegebenenfalls weitere Hilfsstoffe zugesetzt werden. Geeignet sind Hilfsstoffe wie Wasser, isotonische wässrige Kochsalzlösung, Dextrose in Wasser oder Kochsalzlösung, flüssige Glyceryltriester mit niedermolekularen Fettsäuren, Niederalkanole, natürliche Oele wie Maisöl, Erdnussöl, Sesamöl, Castoröl sowie deren Kondensationsprodukte mit Aethlyenoxid und ähnliche, Kohlenwasserstoffe wie Mineralöl pharmazeutischer Qualität, Silicone, Emulgatoren wie Mono- oder Diglyceride von Fettsäuren, Phosphatidsäurederivate wie Lecithin oder Kephalin, Polyalkylenglycole wie Polyäthylenglycol, wässrige Phasen versetzt mit einem Quellmittel wie Natriumcarboxymethylcellulose, Natriumalginat oder Polyvinylpolypyrrolidon, denen noch Dispersionsmittel oder Emulgatoren wie Lecithin zugesetzt sein können, Polyoxyäthylen und ähnliche. Diese Hilfsstoffe können ferner weitere Zusätze wie Konservierungsmittel, Stabilisatoren, Benetzungsmittel und Emulgatoren enthalten.

Die von der Haftklebeschicht b) abziehbare Schutzfolie c) wird vor der Applikation entfernt. Sie besteht aus Materialien, die für die Bestandteile der Haftklebeschicht b) undurchlässig sind. Dabei lassen sich dieselben Materialien, die zur Herstellung der Deckschicht a) dienen können, sowie Metallfolien, z.B. dünne Aluminiumfolie, verwenden. Organische polymere werden durch geeignete Oberflächenbehandlung, z.B. Siliconbehandlung, von der Haftklebeschicht b) abziehbar gemacht.

Die vorliegende Erfindung betrifft bevorzugt ein transdermales therapeutisches System in Form eines Pflasters enthaltend mindestens drei Schichten:

a) eine für die Bestandteile der Haftklebeschicht b) undurchlässige Deckschicht;

b) eine Wirkstoff-abgabefähige Haftklebeschicht aus verschiedenen Polyisobutylenen mit unterschiedlichen Molekulargewichtsbereichen von $1{,}0 \times 10^3$ bis $5{,}0 \times 10^4$ und $1{,}0 \times 10^5$ bis $5{,}0 \times 10^6$, welche einen permeationsfähigen Wirkstoff, eine Kombination aus 5-9,5 Gewichtsteilen 1,8-Cineol und entsprechend 0,5-5 Gewichtsteilen N-Methyl-2-pyrrolidon und gegebenenfalls weitere pharmazeutische Hilfsstoffe enthält, und

c) eine von der Haftklebeschicht b) abziehbare Schutzfolie.

Die vorliegende Erfindung betrifft vor allem ein transdermales therapeutisches System in Form eines Pflasters für die transdermale Verabreichung von Formoterol enthaltend mindestens drei Schichten:

a) eine für die Bestandteile der Haftklebeschicht b) undurchlässige Deckschicht;

b) eine Wirkstoff-abgabefähige Haftklebeschicht aus verschiedenen Polyisobutylenen mit unterschiedlichen Molekulargewichtsbereichen von $1{,}0 \times 10^3$ bis $5{,}0 \times 10^4$ und $1{,}0 \times 10^5$ bis $5{,}0 \times 10^6$, welche Nitroglycerin, Scopolamin, Formoterol, 17β-Oestradiol, Progesteron, 17β-Oestradiol kombiniert mit Norethisteronacetat oder Progesteron oder Lidocain als permeationsfähige Wirkstoffe oder Wirkstoffkombinationen, eine Kombination aus 9 Gewichtsteilen 1,8-Cineol und einem Gewichtsteil N-Methyl-2-pyrrolidon und gegebenenfalls weitere pharmazeutische Hilfsstoffe enthält, und

c) eine von der Haftklebeschicht b) abziehbare Schutzfolie.

Das transdermale therapeutische System gemäss vorliegender Erfindung wird hergestellt, indem man auf der abziehbaren Schutzfolie c) die Haftklebeschicht b) mit ihren Bestandteilen und darauf die Deckschicht a) aufträgt oder indem man umgekehrt verfährt und auf der Deckschicht a) die Haftklebeschicht b) und darauf die Schutzfolie c) aufträgt und nach Durchführung einer der beiden Verfahrensvarianten die verschiedenen Schichten miteinander verbindet und das Pflaster in die gewünschte Form bringt. Dabei wird das Pflaster aus der Vorlage ausgestanzt. Gegebenenfalls wird die Haftklebeschicht b) unter Verwendung von zusätzlichem Klebstoff mit der Deckschicht a) verbunden. Ebenso können die verschiedenen Schichten heiss verschweisst werden.

Die Herstellungsverfahren und Anwendungen sind in den U.S. Patentschriften Nr. 3,598,122, 3,598,123, 3,797,494 und 4,060,084 beschrieben, vorzugsweise in der DE-A-26 04 718 und DE-A-32 05 258 bzw. in den U.S. Patentschriften 4,031,894 und 4,262,003 oder in der Publikation von H. Asche in Schweiz. Rundschau Med. (Praxis) 74, Nr. 11, 257-260 (1985) für die Herstellung von Matrix- oder Monolith-Systemen, wobei die erfindungsgemässe Anwendung nicht auf die in diesen Publikationen beschriebenen transdermalen therapeutischen Systeme beschränkt ist.

Beispiel 1:

a) Herstellung der Haftklebeschicht

Die Bestandteile der Haftklebeschicht werden jeweils in Mengen von 30 g durch Vermischen mit einem Knetwerk, welches hochviskose Pasten verarbeiten kann, z.B. vom Typ EAV 153, Brabender, bei 60° Arbeitstemperatur, zu einer einheitlichen Masse formuliert. Man lässt 7,5 g Polyisobutylen mit einem durchschnittlichen Molekulargewicht von ca. $1,3 \times 10^6$ vom Typ Oppanol® (BASF) B-100 fünf Minuten lang durchkneten und setzt 7,5 g Polyisobutylen mit einem durchschnittlichen Molekulargewicht von ca. $4,0 \times 10^4$ vom Typ OPPANOL B-10 hinzu. Das viskose Gemisch wird fünf Minuten lang geknetet. Anschliessend setzt man eine Lösung von 60 mg Formoterol in 1,5 g N-Methyl-2-pyrrolidon und 13,5 g 1,8-Cineol unter fakultativer Zugabe eines Antioxydationsmittels vom Typ Irganox® (Ciba-Geigy) 1010 hinzu und verarbeitet 30 Minuten lang weiter. Die Formulierung wird nach ihrem Abkühlen auf mögliche Gewichtsverluste gewogen.

b) Herstellung des Monolithsystems

Mit Hilfe einer heizbaren Laborpresse vom Typ Jauch lassen sich bei einer Arbeitstemperatur von 60-80°C Monolithsysteme herstellen. Dabei werden jeweils 3,2 g der gemäss Beispiel 1a) hergestellten Formulierung mit Formoterolbase auf eine Folie aus aluminisiertem Polyester aufgetragen, welche in einen Metallrahmen mit 3 x 8 cm Oeffnung und einer Stärke von 0,1-1,0 mm gespannt ist.

Man deckt mit einer Folie aus silikonisiertem Polyester ab und verbindet das so gebildete Laminat durch Druck von ca. 6 bar etwa dreissig Sekunden lang bei Temperaturen unter 60°C. Anschliessend kann man Pflaster geeigneter Grösse, z.B. von ca. 2,6 cm Durchmesser, aus dem Laminat ausstanzen.

Zur Herstellung von Pflastern mit besonders dünner Haftklebeschicht (weniger als 50 µm Stärke) werden die Bestandteile der Haftklebeschicht in Heptan gelöst und die Lösung auf die Deckschicht aus aluminisiertem Polyester so aufgesprüht, dass nach Verdampfen des Lösungsmittels eine Monolithschicht der gewünschten Stärke resultiert. Die übrigen Verfahrensschritte sind analog.

Beispiel 2:

Versuchsbericht

Ein ca. $3 \times 3$ cm$^2$ grosses Stück Epidermis vom Schwein wurde in einer Diffusionszelle nach der von Franz T.J. J. Invest. Dermatol. 64, 190-195 (1975) angegebenen Methode montiert. Im unteren Akzeptorkompartiment wurde isotonische Phosphatpufferlösung vom pH 7,4 und im oberen Donorkompartiment das transdermale therapeutische System gemäss Beispiel 1 in direkten Kontakt mit der zwischen Donor- und Akzeptorkompartiment eingespannten Schweinehaut (ca. 5 cm$^2$) gebracht. Aus dem Akzeptorkompartiment wird nach 24 Stunden ein Aliquot der wässrigen Akzeptorlösung auf den Gehalt von Formoterol untersucht und daraus die Durchflussgeschwindigkeit des Formoterols [ng/cm$^2 \cdot$h] innerhalb der Zeiträume von 0-4 Stunden, 7-24 Stunden und 24-48 Stunden sowie die gesamten vom System abgegebenen Mengen (= kumulierte Durchflussmengen) [µg/cm$^2$] nach jeweils 24 und 48 Stunden berechnet.

| t [h] | Durchflussgeschwindigkeit [$\mu g/cm^2 \cdot h$] |
|---|---|
| 0 - 4 | 74 |
| 7 - 24 | 309 |
| 2 - 48 | 1026 |

| t | kumulierte Durchflussmenge [$\mu g/cm^2$] |
|---|---|
| 24 | 5,8 |
| 48 | 30,3 |

Beispiel 3:

Analog dem in Beispiel 1a) und 1b) beschriebenen Verfahren lässt sich ein Laminat herstellen, dessen Haftklebeschicht folgende Komponenten enthält:

| | |
|---|---|
| Polyisobutylen OPPANOL B-100 | 6,30 g |
| Polyisobutylen OPPANOL B-10 | 10,00 g |
| N-Methyl-2-pyrrolidon | 0,90 g |
| 1,8-Cineol | 7,80 g |
| 17β-Oestradiol | 0,12 g |
| Progesteron | 0,25 g |
| | 25,37 g |

Es werden scheibenförmige Pflaster von ca. 2,8 cm Durchmesser aus dem Laminat ausgestanzt.

Beispiel 4:

a) Analog dem in Beispiel 1a) und 1b) beschriebenen Verfahren lässt sich ein Laminat herstellen, dessen Haftklebeschicht folgende Komponenten enthält:

| | |
|---|---|
| Polyisobutylen OPPANOL B-100 | 6,30 g |
| Polyisobutylen OPPANOL B-10 | 10,00 g |
| N-Methyl-2-pyrrolidon | 0,90 g |
| 1,8-Cineol | 7,80 g |
| 17β-Oestradiol | 0,12 g |
| | 25,12 g |

b) Analog dem in Beispiel 2) beschriebenen Verfahren wird für das Matrixsystem die Durchflussgeschwindigkeit des 17β-Oestradiols innerhalb der Zeiträume von 0-6, 6-24 und 24-48 Stunden gemessen:

| t [h] | Durchflussgeschwindigkeit [µg/cm² · h] |
|-------|----------------------------------------|
| 0 - 6 | 1,02 |
| 6 - 24 | 0,82 |
| 24 - 48 | 0,58 |

Beispiel 5:

a) Analog dem in Beispiel 1a) und 1b) beschriebenen Verfahren wird ein Laminat hergestellt, dessen Haftklebeschicht folgende Komponenten enthält:

| | |
|---|---|
| Polyisobutylen OPPANOL B-100 | 6,28 g |
| Polyisobutylen OPPANOL B-10 | 9,97 g |
| 1,8-Cineol | 7,87 g |
| N-Methyl-2-pyrrolidon | 0,88 g |
| Progesteron | 0,25 g |
| | 25,25 g |

Es werden scheibenförmige Pflaster von ca. 2,8 cm Durchmesser aus dem Laminat augestanzt.

b) Analog dem in Beispiel 2) beschriebenen Verfahren wird für das Matrixsystem die Durchflussgeschwindigkeit des Progesterons innerhalb der Zeiträume von 0-4, 4-7, 7-24 und 24-48 Stunden gemessen:

| t [h] | Durchflussgeschwindigkeit [µg/cm² · h] |
|-------|----------------------------------------|
| 0 - 4 | 3,09 |
| 4 - 7 | 3,27 |
| 7 - 24 | 2,51 |
| 24 - 48 | 2,08 |

Beispiel 6:

a) Herstellung der Haftklebeschicht

Die Bestandteile der Haftklebeschicht werden jeweils in Mengen von 300 g durch Vermischen in einem Kneter z.B. Brabender PLE 651 verarbeitet. Bei einer Temperatur von 60° wird 60,2 g Polyisobutylen mit einem durchschnittlichen Molekulargewicht von ca. $1,3 \times 10^6$ vom Typ OPPANOL B-100 geknetet. Nach 5 Minuten Knetzeit fügt man 76,0 g Polyisobutylen mit einem durchschnittlichen Molekulargewicht von ca. $4,0 \times 10^4$ vom Typ OPPANOL B-10 hinzu. Man lässt wiederum 5 Minuten kneten und setzt 66,4 g Polyisobutylen mit einem durchschnittlichen Molekulargewicht von ca. $8,0 \times 10^2$ vom Typ OPPANOL B-3 hinzu. Dieses Gemisch wird 5 Minuten geknetet. Anschliessend fügt man eine Lösung von 30 g Lidocain-Base in 5,5 g N-Methyl-2-pyrrolidon, 49,1 g 1,8-Cineol und 12,8 g Paraffinöl hinzu und verarbeitet das Ganze während 25 Minuten zu einer homogenen Masse.

b) Herstellung der Monolithsysteme

Analog Beispiel 1 werden Pflaster in verschiedenen Grössen z.B. 14 cm$^2$, 26 cm$^2$, 37 cm$^2$ und 85 cm$^2$ hergestellt. Als Abdeckfolie wird z.B. eine Polyesterfolie verwendet.

**Patentansprüche**

**Patentansprüche für folgende Vertragsstaaten: AT, BE, CH, DE, FR, GB, IT, LI, LU, NL, SE**

1. Transdermales therapeutisches System in Form eines mehrschichtigen Pflasters mit folgenden Bestandteilen:
   a) eine für die Bestandteile der Haftklebeschicht b) undurchlässige Deckschicht;
   b) eine Wirkstoff-abgabefähige Haftklebeschicht aus permeablem, hautverträglichem, polymerem Material, welche mindestens einen permeationsfähigen Wirkstoff, eine Kombination aus chemisch reinem 1,8-Cineol (Reinheitsgrad höher als 99 %) und N-Methyl-2-pyrrolidon als zusätzliches Hautpenetrationsmittel und gegebenenfalls weitere pharmazeutische Hilfsstoffe enthält, und
   c) eine von der Haftklebeschicht b) abziehbare Schutzfolie.
2. Transdermales therapeutisches System gemäss Anspruch 1, dadurch gekennzeichnet, dass die Kombination aus 1,8-Cineol und N-Methyl-2-pyrrolidon-5-9,5 Gewichtsteile 1,8-Cineol und entsprechend 5-0,5 Gewichtsteile (bezogen auf 10) N-Methyl-2-pyrrolidon enthält.
3. Transdermales therapeutisches System gemäss Anspruch 2, dadurch gekennzeichnet, dass die Kombination aus 1,8-Cineol und N-Methyl-2-pyrrolidon-9 Gewichtsteile 1,8-Cineol und 1 Gewichtsteil N-Methyl-2-pyrrolidon enthält.
4. Transdermales therapeutisches System gemäss einem der Ansprüche 1-3, dadurch gekennzeichnet, dass die Haftklebeschicht b) Nitroglycerin, Scopolamin, Formoterol, 17β-Oestradiol, Progesteron oder Lidocain als permeationsfähigen Wirkstoff enthält.
5. Transdermales therapeutisches System gemäss einem der Ansprüche 1-3, dadurch gekennzeichnet, dass die Haftklebeschicht b) Kombinationen von 17β-Oestradiol mit Norethisteron-17-acetat oder von Progesteron mit 17β-Oestradiol als permeationsfähige Wirkstoffe enthält.
6. Transdermales therapeutisches System gemäss einem der Ansprüche 1-5, dadurch gekennzeichnet, dass die Haftklebeschicht b) ein Gemisch aus verschiedenen Polyisobutylenen mit unterschiedlichen Molekulargewichtsbereichen von $1,0 \times 10^3$ bis $5,0 \times 10^4$ und $1,0 \times 10^5$ bis $5,0 \times 10^6$ als permeables, hautverträgliches, polymeres Material enthält.
7. Transdermales therapeutisches System gemäss einem der Ansprüche 1-6 bestehend aus mindestens drei Schichten:
   a) eine für die Bestandteile der Haftklebeschicht b) undurchlässige Deckschicht;
   b) eine Wirkstoff-abgabefähige Haftklebeschicht aus verschiedenen Polyisobutylenen mit unterschiedlichen Molekulargewichtsbereichen von $1,0 \times 10^3$ bis $5,0 \times 10^4$ und $1,0 \times 10^5$ bis $5,0 \times 10^6$, welche Nitroglycerin, Scopolamin, Formoterol, 17β-Oestradiol, Progesteron, 17β-Oestradiol kombiniert mit Norethisteronacetat oder Progesteron oder Lidocain als permeationsfähige Wirkstoffe oder Wirkstoffkombinationen, eine Kombination aus 9 Gewichtsteilen chemisch reinem 1,8-Cineol und einem Gewichtsteil N-Methyl-2-pyrrolidon und gegebenenfalls weitere pharmazeutische Hilfsstoffe enthält, und
   c) eine von der Haftklebeschicht b) abziehbare Schutzfolie
8. Verfahren zur Herstellung eines pharmazeutischen Pflasters gemäss Anspruch 1, dadurch gekennzeichnet, dass man auf der abziehbaren Schutzfolie c) die Haftklebeschicht b) mit ihren Bestandteilen und darauf die Deckschicht a) aufträgt oder umgekehrt verfährt und auf der Deckschicht a) die Haftklebeschicht b) und darauf die Schutzfolie c) aufträgt und nach Durchführung einer der beiden Verfahrensvarianten die Schichten miteinander verbindet und das Pflaster in die gewünschte Form bringt.
9. Pharmazeutisches Pflaster gemäss Anspruch 1, zur transdermalen Anwendung in einem Verfahren zur therapeutischen Behandlung des menschlichen oder tierischen Körpers.
10. Verwendung des Gemisches aus aus chemisch reinem 1,8-Cineol und N-Methyl-2-pyrrolidon zur Herstellung eines transdermalen therapeutischen Systems gemäss Anspruch 1.
11. Verwendung gemäss Anspruch 10 des Gemisches aus 9 Gewichtsteilen 1,8-Cineol und einem Gewichtsteil N-Methyl-2-pyrrolidon zur Herstellung eines transdermalen therapeutischen System gemäss Anspruch 7.

**Patentansprüche für folgende Vertragsstaaten: ES, GR**

1. Verfahren zur Herstellung eines transdermalen therapeutischen Systems in Form eines mehrschichtigen Pflasters mit folgenden Bestandteilen:

a) eine für die Bestandteile der Haftklebeschicht b) undurchlässige Deckschicht;

b) eine Wirkstoff-abgabefähige Haftklebeschicht aus permeablem, hautverträglichem, polymerem Material, welche mindestens einen permeationsfähigen Wirkstoff, eine Kombination aus chemisch reinem 1,8-Cineol (Reinheitsgrad höher als 99 %) und N-Methyl-2-pyrrolidon als zusätzliches Hautpenetrationsmittel und gegebenenfalls weitere pharmazeutische Hilfsstoffe enthält, und

c) eine von der Haftklebeschicht b) abziehbare Schutzfolie, dadurch gekennzeichnet, dass man auf der abziehbaren Schutzfolie c) die Haftklebeschicht b) mit ihren Bestandteilen und darauf die Deckschicht a) aufträgt oder umgekehrt verfährt und auf der Deckschicht a) die Haftklebeschicht b) und darauf die Schutzfolie c) aufträgt und nach Durchführung einer der beiden Verfahrensvarianten die Schichten miteinander verbindet und das Pflaster in die gewünschte Form bringt.

2. Verfahren zur Herstellung eines transdermalen therapeutischen Systems gemäss Anspruch 1, dadurch gekennzeichnet, dass die Kombination aus 1,8-Cineol und N-Methyl-2-pyrrolidon 5-9,5 Gewichtsteile 1,8-Cineol und entsprechend 5-0,5 Gewichtsteile (bezogen auf 10) N-Methyl-2-pyrrolidon enthält.

3. Verfahren zur Herstellung eines transdermalen therapeutischen Systems gemäss Anspruch 2, dadurch gekennzeichnet, dass die Kombination aus 1,8-Cineol und N-Methyl-2-pyrrolidon 9 Gewichtsteile 1,8-Cineol und 1 Gewichtsteil N-Methyl-2-pyrrolidon enthält.

4. Verfahren zur Herstellung eines transdermalen therapeutischen Systems gemäss einem der Ansprüche 1-3, dadurch gekennzeichnet, dass die Haftklebeschicht b) Nitroglycerin, Scopolamin, Formoterol, 17β-Oestradiol, Progesteron oder Lidocain als permeationsfähigen Wirkstoff enthält.

5. Verfahren zur Herstellung eines transdermalen therapeutischen Systems gemäss einem der Ansprüche 1-3, dadurch gekennzeichnet, dass die Haftklebeschicht b) Kombinationen von 17β-Oestradiol mit Norethisteron-17-acetat oder von Progesteron mit 17β-Oestradiol als permeationsfähige Wirkstoffe enthält.

6. Verfahren zur Herstellung eines transdermalen therapeutischen Systems gemäss einem der Ansprüche 1-5, dadurch gekennzeichnet, dass die Haftklebeschicht b) ein Gemisch aus verschiedenen Polyisobutylenen mit unterschiedlichen Molekulargewichtsbereichen von $1,0 \times 10^3$ bis $5,0 \times 10^4$ und $1,0 \times 10^5$ bis $5,0 \times 10^6$ als permeables, hautverträgliches, polymeres Material enthält.

7. Verfahren zur Herstellung eines transdermalen therapeutischen Systems gemäss einem der Ansprüche 1-6 bestehend aus mindestens drei Schichten:

a) eine für die Bestandteile der Haftklebeschicht b) undurchlässige Deckschicht;

b) eine Wirkstoff-abgabefähige Haftklebeschicht aus verschiedenen Polyisobutylenen mit unterschiedlichen Molekulargewichtsbereichen von $1,0 \times 10^3$ bis $5,0 \times 10^4$ und $1,0 \times 10^5$ bis $5,0 \times 10^6$, welche Nitroglycerin, Scopolamin, Formoterol, 17β-Oestradiol, Progesteron, 17β-Oestradiol kombiniert mit Norethisteronacetat oder Progesteron oder Lidocain als permeationsfähige Wirkstoffe oder Wirkstoffkombinationen, eine Kombination aus 9 Gewichtsteilen chemisch reinem 1,8-Cineol und einem Gewichtsteil N-Methyl-2-pyrrolidon und gegebenenfalls weitere pharmazeutische Hilfsstoffe enthält, und

c) eine von der Haftklebeschicht b) abziehbare Schutzfolie, dadurch gekennzeichnet, dass man die in Anspruch 1 genannten Massnahmen ausführt.

**Claims**

**Claims for the following Contracting States: AT, BE, CH, DE, FR, GB, IT, LI, LU, NL, SE**

1. A transdermal therapeutic system in the form of a multi-layered plaster comprising the following components:

a) a backing layer which is impermeable to the components of the adhesive layer b),

b) an adhesive layer capable of drug release and consisting of a permeable polymeric material which is compatible with the skin and which contains at least one drug that is capable of permeation, a combination of chemically pure 1,8-cineole (degree of purity greater than 99 %) and N-methyl-2-pyrrolidone as additional skin penetration agent and optionally further pharmaceutical excipients, and

c) a protective film which can be peeled from the adhesive layer b).

2. A transdermal therapeutic system according to claim 1, wherein the combination of 1,8-cineole and N-methyl-2-pyrrolidone comprises 5 to 9.5 parts by weight of 1,8-cineole and, accordingly, 5 to 0.5 parts by weight (based on 10 parts by weight) of N-methyl-2-pyrrolidone.

3. A transdermal therapeutic system according to claim 2, wherein the combination of 1,8-cineole and N-methyl-2-pyrrolidone comprises 9 parts by weight of 1,8-cineole and 1 part by weight of N-methyl-2-pyrrolidone.

4. A transdermal therapeutic system according to any one of claims 1 to 3, wherein the adhesive layer b) contains nitroglycerin, scopolamine, formoterol, 17β-oestradiol, progesterone or lidocaine as drug capable of permeation.

5. A transdermal therapeutic system according to any one of claims 1 to 3, wherein the adhesive layer b) contains a combination of 17β-oestradiol with norethisterone-17-acetate or of progesterone with 17β-oestradiol as drugs capable of permeation.

6. A transdermal therapeutic system according to any one of claims 1 to 5, wherein the adhesive layer b) comprises a mixture of different polyisobutylenes having different molecular weight ranges of $1.0 \times 10^3$ to $5.0 \times 10^4$ and $1.0 \times 10^5$ to $5.0 \times 10^6$ as permeable polymeric material that is compatible with the skin.

7. A transdermal therapeutic system according to any one of claims 1 to 6, comprising at least three layers:
a) a backing layer which is impermeable to the components of the adhesive layer b),
b) an adhesive layer capable of drug release and consisting of different polyisobutylenes having different molecular weight ranges of $1.0 \times 10^3$ to $5.0 \times 10^4$ and $1.0 \times 10^5$ to $5.0 \times 10^6$, which layer contains nitroglycerin, scopolamine, formoterol, 17β-oestradiol, progesterone, 17β-oestradiol in combination with norethisterone acetate or progesterone, or lidocaine as drug or drug combination capable of permeation, a combination of 9 parts by weight of chemically pure 1,8-cineole and 1 part by weight of N-methyl-2-pyrrolidone and optionally further pharmaceutical excipients, and
c) a protective film which can be peeled from the adhesive layer b).

8. A process for the preparation of a pharmaceutical plaster according to claim 1, which comprises applying the adhesive layer b) with its components to the removable protective film c) and then applying the backing layer a) or, conversely, applying the adhesive layer b) to the backing layer a) and then applying the protective film c) and, after carrying out one of these two variants, combining the layers and bringing the plaster into the desired form.

9. A pharmaceutical plaster according to claim 1 for transdermal application in a method for the therapeutic treatment of the human or animal body.

10. Use of a mixture of chemically pure 1,8-cineole and N-methyl-2-pyrrolidone for the preparation of a transdermal therapeutic system according to claim 1.

11. Use according to claim 10 of a mixture of 9 parts by weight of 1,8-cineole and 1 part by weight of N-methyl-2-pyrrolidone for the preparation of a transdermal therapeutic system according to claim 7.

**Claims for the following Contracting States: ES, GR**

1. A process for the preparation of a transdermal therapeutic system in the form of a multi-layered plaster comprising the following components:
a) a backing layer which is impermeable to the components of the adhesive layer b),
b) an adhesive layer capable of drug release and consisting of a permeable polymeric material which is compatible with the skin and which contains at least one drug that is capable of permeation, a combination of chemically pure 1,8-cineole (degree of purity greater than 99 %) and N-methyl-2-pyrrolidone as additional skin penetration agent and optionally further pharmaceutical excipients, and
c) a protective film which can be peeled from the adhesive layer b), which process comprises applying the adhesive layer b) with its components to the removable protective film c) and then applying the backing layer a) or, conversely, applying the adhesive layer b) to the backing layer a) and then applying the protective film c) and, after carrying out one of these two variants, combining the layers and bringing the plaster into the desired form.

2. A process for the preparation of a transdermal therapeutic system according to claim 1, wherein the combination of 1,8-cineole and N-methyl-2-pyrrolidone comprises 5 to 9.5 parts by weigth of 1,8-cineole and, accordingly, 5 to 0.5 parts by weight (based on 10 parts by weight) of N-methyl-2-pyrrolidone.

3. A process for the preparation of a transdermal therapeutic system according to claim 2, wherein the combination of 1,8-cineole and N-methyl-2-pyrrolidone comprises 9 parts by weight of 1,8-cineole and 1 part by weight of N-methyl-2-pyrrolidone.

4. A process for the preparation of a transdermal therapeutic system according to any one of claims 1 to 3, wherein the adhesive layer b) contains nitroglycerin, scopolamine, formoterol, 17β-oestradiol, progesterone or lidocaine as drug capable of permeation.

5. A process for the preparation of a transdermal therapeutic system according to any one of claims 1 to 3, wherein the adhesive layer b) contains a combination of 17β-oestradiol with norethisterone-17-acetate or of progesterone with 17β-oestradiol as drugs capable of permeation.

6. A process for the preparation of a transdermal therapeutic system according to any one of claims 1 to 5, wherein the adhesive layer b) comprises a mixture of different polyisobutylenes having different molecular weight ranges of $1.0 \times 10^3$ to $5.0 \times 10^4$ and $1.0 \times 10^5$ to $5.0 \times 10^6$ as permeable polymeric material that is compatible with the skin.

7. A process for the preparation of a transdermal therapeutic system according to any one of claims 1 to 6, comprising at least three layers:

a) a backing layer which is impermeable to the components of the adhesive layer b),

b) an adhesive layer capable of drug release and consisting of different polyisobutylenes having different molecular weight ranges of $1.0 \times 10^3$ to $5.0 \times 10^4$ and $1.0 \times 10^5$ to $5.0 \times 10^6$, which layer contains nitroglycerin, scopolamine, formoterol, 17β-oestradiol, progesterone, 17β-oestradiol in combination with norethisterone acetate or progesterone, or lidocaine as drug or drug combination capable of permeation, a combination of 9 parts by weight of chemically pure 1,8-cineole and 1 part by weight of N-methyl-2-pyrrolidone and optionally further pharmaceutical excipients, and

c) a protective film which can be peeled from the adhesive layer b), which process comprises carrying out the measures mentioned in claim 1.

## Revendications

### Revendications pour les Etats contractants suivants: AT, BE, CH, DE, FR, GB, IT, LI, LU, NL, SE

1. Système thérapeutique transdermique sous forme d'un emplâtre multicouche ayant les constituants suivants:

a) une couche de recouvrement imperméable pour les constituants de la couche auto-adhésive b);

b) une couche auto-adhésive apte à libérer l'agent actif et faite de matière polymère perméable, compatible avec la peau, contenant au moins un agent actif apte à la perméation, une combinaison de 1,8-cinéol chimiquement pur (d'un degré de pureté supérieur à 99%) et de N-méthyl-2-pyrrolidone comme agent supplémentaire de pénétration dans la peau et éventuellement d'autres adjuvants pharmaceutiques; et

c) une feuille de protection, arrachable de la couche auto-adhésive b).

2. Système thérapeutique transdermique selon la revendication 1, caractérisé en ce que la combinaison de 1,8-cinéol et de N-méthyl-2-pyrrolidone contient 5-9,5 parties en poids de 1,8-cinéol et en correspondance 5-0,5 parties en poids (par rapport à 10) de N-méthyl-2-pyrrolidone.

3. Système thérapeutique transdermique selon la revendication 2, caractérisé en ce que la combinaison de 1,8-cinéol et de N-méthyl-2-pyrrolidone contient 9 parties en poids de 1,8-cinéol et 1 partie en poids de N-méthyl-2-pyrrolidone.

4. Système thérapeutique transdermique selon l'une quelconque des revendications 1 à 3, caractérisé en ce que la couche auto-adhésive b) contient de la nitroglycérine, de la scopolamine, du formotérol, du 17β-oestradiol, de la progestérone ou de la lidocaïne en tant qu'agent actif apte à la perméation.

5. Système thérapeutique transdermique selon l'une quelconque des revendications 1 à 3, caractérisé en ce que la couche auto-adhésive b) contient des combinaisons de 17 - oestradiol avec du 17-acétate de noréthistérone ou de progestérone avec du 17β-oestradiol en tant qu' agent actif apte à la perméation.

6. Système thérapeutique transdermique selon l'une quelconque des revendications 1 à 5, caractérisé en ce que la couche auto-adhésive b) contient un mélange de différents polyisobutylènes ayant différents domaines de masses moléculaires de $1,0 \times 10^3$ à $5,0 \times 10^4$ et de $1,0 \times 10^5$ à $5,0 \times 10^6$, en tant que matière polymère perméable, compatible avec la peau.

7. Système thérapeutique transdermique selon l'une quelconque des revendications 1 à 6, consistant en au moins trois couches:

a) une couche de recouvrement imperméable pour les constituants de la couche auto-adhésive b);

b) une couche auto-adhésive apte à libérer l'agent actif et faite de différents polyisobutylènes ayant différents domaines de masses moléculaires de $1,0 \times 10^3$ à $5,0 \times 10^4$ et $1,0 \times 10^5$ à $5,0 \times 10^6$, contenant de la nitroglycérine, de la scopolamine, du formotérol, du 17β-oestradiol, de la progestérone, du 17β-oestradiol combiné avec de l'acétate de noréthistérone ou de la progestérone ou de la lidocaïne en tant qu'agents actifs aptes à la perméation ou combinaisons d'agents actifs aptes à la perméation, une combinaison de 9 parties en poids de 1,8-cinéol chimiquement pur et 1 partie en poids de N-méthyl-2-pyrrolidone et éventuellement d'autres adjuvants pharmaceutiques; et

c) une feuille de protection, arrachable de la couche auto-adhésive b).

8. Procédé pour la préparation d'un emplâtre pharmaceutique selon la revendication 1, caractérisé en ce qu'on place sur la feuille pelable c) la couche auto-adhésive b) avec ses constituants et au-dessus la couche de recouvrement a) ou bien à l'opposé on dispose sur la couche de recouvrement a) la couche auto-adhésive b) et au-dessus la feuille de protection c) et, une fois réalisée l'une des deux variantes opératoires, on réunit entre elles les différentes couches et l'on met l'emplâtre sous la forme souhaitée.

9. Emplâtre pharmaceutique selon la revendication 1, pour l'application transdermique dans un procédé pour le traitement thérapeutique du corps humain ou animal,

10. Application du mélange de 1,8-cinéol chimiquement pur et de N-méthyl-2-pyrrolidone pour la production d'un système thérapeutique transdermique selon la revendication 1.

11. Application selon la revendication 10 du mélange de 9 parties en poids de 1,8-cinéol et de 1 partie en poids de N-méthyl-2-pyrrolidone pour la production d'un système thérapeutique transdermique selon la revendication 7.

**Revendications pour les Etats contractants suivants: ES, GR**

1. Procédé pour la préparation d'un système thérapeutique transdermique sous la forme d'un emplâtre multicouche ayant les constituants suivants:

a) une couche de recouvrement imperméable pour les constituants de la couche auto-adhésive b);

b) une couche auto-adhésive apte à libérer l'agent actif et faite de matière polymère perméable, compatible avec la peau, contenant au moins un agent actif apte à la perméation, une combinaison de 1,8-cinéol chimiquement pur (d'un degré de pureté supérieur à 99%) et de N-méthyl-2-pyrrolidone comme agent supplémentaire de pénétration dans la peau et éventuellement d'autres adjuvants pharmaceutiques; et

c) une feuille de protection, arrachable de la couche auto-adhésive b), caractérisé en ce qu'on place sur la feuille pelable c) la couche auto-adhésive b) avec ses constituants et au-dessus la couche de recouvrement a) ou bien à l'opposé on dispose sur la couche de recouvrement a) la couche auto-adhésive b) et au-dessus la feuille de protection c) et, une fois réalisée l'une des deux variantes opératoires, on réunit entre elles les différentes couches et l'on met l'emplâtre sous la forme souhaitée,

2. Procédé pour la préparation d'un système thérapeutique transdermique selon la revendication, caractérisé en ce que la combinaison de 1,8-cinéol et de N-méthyl-2-pyrrolidone contient 5-9,5 parties en poids de 1,8-cinéol et en correspondante 5-0,5 parties en poids (par rapport à 10) de N-méthyl-2-pyrrolidone.

3. Procédé pour la préparation d'un système thérapeutique transdermique selon la revendication 2, caractérisé en ce que la combinaison de 1,8-cinéol et de N-méthyl-2-pyrrolidone contient 9 parties en poids de 1,8-cinéol et 1 partie en poids de N-méthyl-2-pyrrolidone.

4. Procédé pour la préparation d'un système thérapeutique transdermique selon l'une quelconque des revendications 1 à 3, caractérisé en ce que la couche auto-adhésive b) contient de la nitroglycérine, de la scopolamine, du formotérol, du 17β-oestradiol, de la progestérone ou de la lidocaïne en tant qu'agent actif apte à la perméation.

5. Procédé pour la préparation d'un système thérapeutique transdermique selon l'une quelconque des revendications 1 à 3, caractérisé en ce que la couche auto-adhésive b) contient des combinaisons de 17β-oestradiol avec du 17-acétate de noréthistérone ou de progestérone avec du 17β-oestradiol en tant qu'agent actif apte à la perméation.

6. Procédé pour la préparation d'un système thérapeutique transdermique selon l'une quelconque des revendications 1 à 5, caractérisé en ce que la couche auto-adhésive b) contient un mélange de différents polyisobutylènes ayant différents domaines de masses moléculaires de $1,0 \times 10^3$ à $5,0 \times 10^4$ et $1,0 \times 10^5$ à $5,0 \times 10^6$, en tant que matière polymère perméable, compatible avec la peau.

7. Procédé pour la préparation d'un système thérapeutique transdermique selon l'une quelconque des revendications 1 à 6, consistant en au moins trois couches:

a) une couche de recouvrement imperméable pour les constituants de la couche auto-adhésive b);

b) une couche auto-adhésive apte à libérer l'agent actif et faite de différents polyisobutylènes ayant différents domaines de masses moléculaires de $1,0 \times 10^3$ à $5,0 \times 10^4$ et $1,0 \times 10^5$ à $5,0 \times 10^6$, contenant de la nitroglycérine, de la scopolamine, du formotérol, du 17β-oestradiol, de la progestérone, du 17-oestradiol combiné avec de l'acétate de noréthistérone ou de la progestérone ou de la lidocaïne en tant qu'agents actifs aptes à la perméation ou combinaisons d'agents actifs aptes à la perméation, une combinaison de 9 parties en poids de 1,8-cinéol chimiquement pur et de 1 partie en poids de N-méthyl-2-pyrrolidone et éventuellement d'autres adjuvants pharmaceutiques; et

c) une feuille de protection, arrachable de la couche auto-adhésive b), caractérisé en ce qu'on exécute les mesures indiquées dans la revendication 1.